# FASCICULE DE BREVET EUROPEEN

(11) **EP 3 649 927 B1**
(45) Date de publication et mention de la délivrance du brevet: **06.10.2021**
(21) Numéro de dépôt: 18205481.7
(22) Date de dépôt: 09.11.2018
(51) Int. Cl.: A61B 5/349, A61B 5/021, A61B 5/00, A61B 5/0285, A61B 5/02, A61B 5/0215, A61B 5/352

(54) **SYSTÈME DE DÉTERMINATION D'UNE VITESSE D'ONDE DE POULS ARTÉRIELLE**
SYSTEM ZUR BESTIMMUNG DER GESCHWINDIGKEIT EINER ARTERIELLEN PULSWELLE
SYSTEM FOR DETERMINING AN ARTERIAL PULSE WAVE VELOCITY

(43) Date de publication de la demande: 13.05.2020
(73) Titulaire: Université Claude Bernard Lyon 1, 69100 Villeurbanne (FR); Hospices Civils De Lyon, 69002 Lyon (FR)
(72) Inventeur: LANTELME, Pierre, 69270 FONTAINES SAINT MARTIN (FR); CIVIDJIAN, Andrei, 69330 MEYZIEU (FR); HARBAOUI, Brahim, 69500 BRON (FR)
(74) Mandataire: INNOV-GROUP

(56) Documents cités:
- EP-A1- 3 251 591
- BRAHIM HARBAOUI ET AL: "Development of Coronary Pulse Wave Velocity: New Pathophysiological Insight Into Coronary Artery Disease", JOURNAL OF THE AMERICAN HEART ASSOCIATION, vol. 6, no. 2, 2 février 2017 (2017-02-02) , pages 1-11, XP055579163, ISSN: 2047-9980, DOI: 10.1161/JAHA.116.004981
- TAEWOO NAM ET AL: "A Coronary Pulse Wave Velocity Measurement System", 2007 ANNUAL INTERNATIONAL CONFERENCE OF THE IEEE ENGINEERING IN MEDICINE AND BIOLOGY SOCIETY : [EMBC '07] ; LYON, FRANCE, 22 - 26 AUGUST 2007 ; [IN CONJUNCTION WITH THE BIENNIAL CONFERENCE OF THE SOCIÉTÉ FRANÇAISE DE GÉNIE BIOLOGIQUE ET MÉDICAL (SFGB, 22 août 2007 (2007-08-22), pages 975-977, XP031336335, ISBN: 978-1-4244-0787-3

## Description

L'invention concerne les systèmes d'assistance en vue d'étudier des pathologies artérielles, par exemple les systèmes permettant d'anticiper un risque de rupture d'une plaque d'athérome à l'intérieur d'une artère coronaire, en vue d'affiner la stratégie de prise en charge d'un patient lors d'un examen de coronarographie, ou des systèmes étudiant des pathologies dans des artères aortiques, rénales, hépatiques, et plus généralement toute artère à risque de rupture de plaque d'athérome et/ou thrombose.

Il est connu que les calcifications d'une artère étaient à l'origine d'une rigidification de cette artère. Différentes techniques sont connues pour estimer la rigidité artérielle: mesure de la pression puisée, estimation des calcifications artérielles, la vitesse de l'onde de pouls, cette dernière technique étant la plus utilisée. Des études ont confirmé par exemple, que la rigidité de l'artère aortique, mesurée par de différentes techniques, est un indicateur améliorant la prédiction des pathologies cardiovasculaires. Une étude médicale a montré que la vitesse d'onde de pouls à l'intérieur d'une artère coronaire est plus basse chez des patients présentant un syndrome coronaire aigu, possiblement dû à une rupture de plaque, que les patients sans cette pathologie.

S'il existe des systèmes permettant de réaliser des mesures de vitesses d'onde de pouls aortiques, et donc de réaliser des études correspondantes, il s'avère encore délicat de déterminer avec précision une vitesse d'onde de pouls coronaire. Les praticiens ont donc des difficultés à mesurer la vitesse d'onde de pouls coronaire et ainsi à déterminer l'impact de la rigidité coronaire sur l'évolution d'une lésion coronaire, comme par exemple le risque de thrombose aigue. Par ailleurs, la détermination de la vitesse d'onde de pouls aortique s'avère insuffisante pour déterminer avec précision les pathologies sur les artères coronaires. En particulier, la mesure de la vitesse d'onde de pouls aortique ne permet pas d'anticiper un risque de rupture d'une plaque intra coronaire.

Le document 'A Coronary Pulse Wave Velocity Measurement System', publié par Messieurs Taewoo Nam et alias, pages 975 à 977 dans Proceedings of the 29th Annual International Conférence of the IEEE EMBS, dans le cadre d'une conférence à la Cité Internationale de Lyon en France du 23 au 26 Août 2007, décrit un exemple de procédé de calcul de la vitesse d'onde de pouls coronaire, uniquement basé sur des calculs manuels, à titre expérimental.

La publication de la demande de brevet EP3251591 décrit un procédé de détermination d'une vitesse d'onde de pouls coronaire, basé sur la durée séparant les fronts montants respectifs, entre pression diastolique et systolique, d'un signal de pression sanguine proximale dans une artère coronaire et d'un signal de pression sanguine distale dans cette artère coronaire. Cette publication propose un procédé améliorant la précision d'identification des fronts montants. Un front montant distal est notamment identifié par un décalage par rapport à un front descendant distal.

En pratique, les fronts montants des signaux de pression sanguine peuvent être difficiles à identifier. Des pics de pression artérielle peuvent en effet apparaître avant les fronts montants de pression. Lorsque de tels pics de pression apparaissent, ils perturbent l'identification des fronts montants et le calcul de la vitesse d'onde de pouls artérielle. Par ailleurs, la rigidité artérielle peut varier entre une phase de compression et une phase de décompression.

Le document "Development of Coronary Pulse Wave Velocity: New Pathophysiological Insight Into Coronary Artery Disease', publié par Brahim HARBAOUI et alias dans Journal of the American heart association, volume 6 N°2, le 2 février 2017 en pages 1-11, décrit un procédé de détermination d'une vitesse d'onde de pouls coronaire, basé sur la durée séparant les fronts montants respectifs, entre pression diastolique et systolique, d'un signal de pression sanguine proximale dans une artère coronaire et d'un signal de pression sanguine distale dans cette artère coronaire. Cette publication propose un procédé améliorant la précision d'identification des fronts montants. Un front montant distal est notamment identifié par un décalage par rapport à un front descendant distal.

L'invention vise à résoudre un ou plusieurs de ces inconvénients. L'invention porte ainsi sur un système de détermination d'une vitesse d'onde de pouls selon la revendication 1.

L'invention porte également sur les variantes des revendications dépendantes.

D'autres caractéristiques et avantages de l'invention ressortiront clairement de la description qui en est faite ci-après, à titre indicatif et nullement limitatif, en référence aux dessins annexés, dans lesquels :
[Fig.1] une représentation schématique d'un cœur et de ses artères coronaires ;
[Fig.2] est une vue en coupe d'un fil-guide selon un aspect de l'invention, inséré dans une artère coronaire comportant une sténose ;
[Fig.3] est une vue en coupe schématique d'un dispositif à fil-guide de FFR (pour Fractional Flow Reserve en langue anglaise, soit la mesure de la réserve coronaire) selon un aspect de l'invention ;
[Fig.4] est une représentation schématique d'un système de traitement de signaux en vue de déterminer la vitesse d'onde de pouls et le caractère ischémiant d'une sténose coronaire selon un aspect de l'invention ;
[Fig.5] est un diagramme illustrant un exemple de cycle de pression artérielle coronaire proximale ;
[Fig.6] est un diagramme illustrant un exemple de cycle de pression artérielle coronaire distale ;
[Fig.7] illustre des paramètres temporels au niveau du front montant d'une pression artérielle coronaire proximale et d'une pression artérielle coronaire distale ;
[Fig.8] illustre un exemple de détermination de paramètres temporels au niveau du front montant d'une pression artérielle coronaire proximale et d'une pression artérielle coronaire distale.

Les inventeurs ont constaté que des pics de pression pouvaient apparaître dans les signaux de pression intra-coronaire mesurés aussi bien en position proximale que distale, préalablement aux fronts montants entre la pression diastolique et la pression systolique. L'interprétation des inventeurs est que de tels pics de pression précoces sont dus à une onde régressive, dans le sens opposé au sens d'écoulement sanguin (c'est-à-dire dans le sens allant de l'extrémité coronaire distale vers extrémité coronaire proximale). De tels pics de pression artérielle sont dus à une pression exercée depuis l'extérieur de l'artère, par exemple par d'autres parties de l'organisme ou par un objet extérieur. L'onde régressive peut par exemple être provoquée par une compression de l'extrémité distale de l'artère coronaire par le myocarde lors de la contraction cardiaque. De façon surprenante, les inventeurs ont identifié que l'analyse de tels pics de pression précoces peut être exploitée pour déterminer la vitesse de l'onde de pouls dans l'artère coronaire.

L'invention propose un système de calcul numérique d'une vitesse d'onde de pouls, basée sur l'analyse de l'onde régressive identifiée. L'invention s'applique en particulier au calcul d'une vitesse d'onde de pouls artérielle, pour laquelle une pression extérieure peut empêcher la détection du front montant de pression avec précision, en particulier une vitesse d'onde de pouls coronaire.

L'invention permet de déterminer précisément et de façon reproductible la vitesse d'onde de pouls, facilitant ainsi une prise de décision du praticien, en vue de déterminer la prise en charge du patient, dans des cas où une onde de pouls régressive détériore la capacité d'analyse des fronts montants des signaux de pression sanguine. De plus, dans le cas d'une artère coronaire, l'invention peut être mise en œuvre en même temps que la procédure d'introduction d'un fil-guide de l'indice de FFR déjà validée cliniquement.

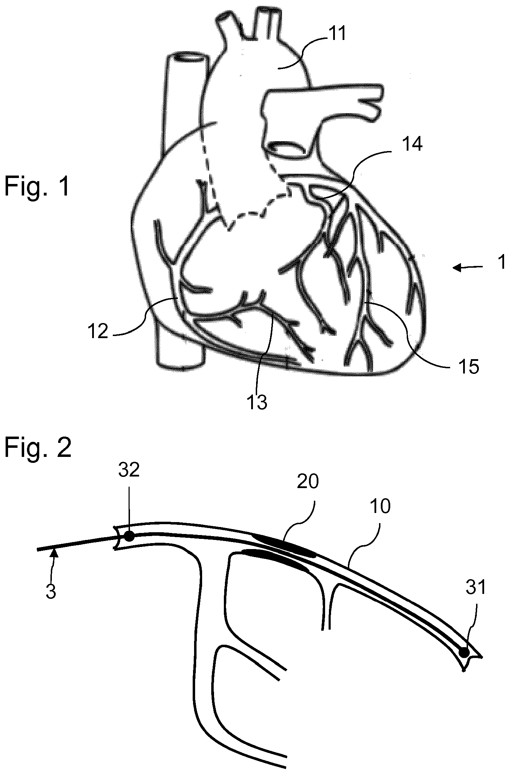

La figure 1 est une représentation schématique d'un cœur humain 1. On distingue l'artère aorte 11 raccordée au cœur, et des artères coronaires 12 à 15. Les artères coronaires sont destinées à alimenter les muscles cardiaques en sang oxygéné. La figure 1 illustre notamment l'artère coronaire droite 12, l'artère coronaire postérieure descendante 13, l'artère coronaire circonflexe gauche 14 et l'artère coronaire antérieure gauche descendante 15. L'invention sera ici décrite dans une application particulière à une artère coronaire mais pourra éventuellement être mise en œuvre avec d'autres types d'artères.

La figure 2 permet d'illustrer un exemple d'un mode de récupération de signaux, en vue de calculer la vitesse d'onde de pouls coronaire d'un patient. Un fil-guide de FFR 3 est introduit de façon à positionner son extrémité libre à l'intérieur d'une artère coronaire 10. Le fil-guide 3 comporte ici deux capteurs de pression 31 et 32 au niveau de son extrémité libre. Les termes distal et proximal se rapporteront à la proximité relative d'un point considéré par rapport au flux sanguin provenant du cœur. Le capteur de pression 31 est en position distale, afin de mesurer la pression sanguine à proximité de l'embranchement de l'artère 10 avec le tissu des capillaires. Le capteur de pression 32 est en position proximale, afin de mesurer la pression sanguine à proximité de l'embranchement de l'artère 10 avec l'artère aortique. Le capteur de pression 32 est décalé par rapport au capteur de pression 31, d'une distance Dmd prédéfinie sur la longueur du fil-guide 3. L'artère coronaire 10 illustrée ici comporte une sténose 20, les capteurs de pression 31 et 32 étant positionnés de part et d'autre de cette sténose 20.

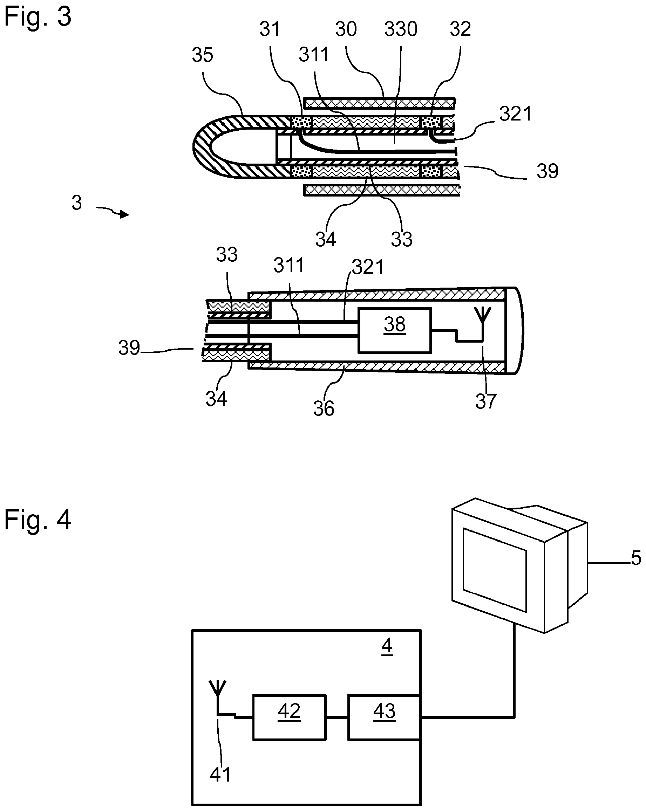

La figure 3 est une vue en coupe schématique de deux extrémités d'un fil-guide 3 utilisable pour la mise en œuvre de l'invention. Le fil-guide 3 comporte un fil 39 coulissant de façon connue en soi dans une gaine externe de stockage 30. Le fil 39 est illustré uniquement de façon schématique pour détailler sa structure, le fil 39 n'est pas illustré à l'échelle. Le fil 39 est flexible pour s'adapter à la morphologie de l'artère coronaire dans lequel il est introduit. Le fil 39 comporte un fourreau métallique creux 33. Le fourreau métallique 33 est recouvert d'une gaine 34 en matériau synthétique. Le fil 39 comporte avantageusement un embout 35 au niveau de son extrémité libre. L'embout 35 peut avantageusement être flexible et radio-opaque. L'embout 35 est ici fixé sur le fourreau métallique 33.

Le capteur de pression 31 est ici fixé à la périphérie du fourreau 33, et positionné entre l'embout 35 et la gaine 34. Le capteur de pression 31 est destiné à mesurer la pression sanguine distale. Le capteur de pression 31 (de structure connue en soi) est connecté à un câble ou à une fibre optique 311 de transmission du signal de pression. Le câble 311 traverse un orifice ménagé dans le fourreau 33 pour sa connexion au capteur 31. Le câble ou la fibre optique 311 s'étend dans un alésage interne 330 du fourreau 33.

Le capteur de pression 32 est ici fixé à la périphérie du fourreau 33, et positionné entre deux tronçons de la gaine 34. Le capteur de pression 32 est destiné à mesurer la pression sanguine proximale. Le capteur de pression 32 est connecté à un câble ou à une fibre optique 321 de transmission du signal de pression. Le câble 321 traverse un orifice ménagé dans le fourreau 33 pour sa connexion au capteur 32. Le câble ou la fibre optique 321 s'étend dans l'alésage interne 330 du fourreau 33.

Le fil 39 est ici flexible mais sensiblement non compressible ou inextensible. Ainsi, le fil 39 maintient ici une distance Dmd constante entre les capteurs 31 et 32. La distance entre les capteurs 31 et 32 correspond en pratique à la distance curviligne entre ces capteurs le long du fil 39. La distance entre les capteurs 31 et 32 est avantageusement au moins égale à 50 mm, de façon à garantir une distance suffisamment élevée entre ces capteurs 31 et 32, afin de disposer d'une bonne précision pour le calcul de la vitesse d'onde de pouls. Par ailleurs, la distance entre les capteurs 31 et 32 est avantageusement au plus égale à 200 mm, de sorte que le fil-guide 3 reste utilisable dans la plupart des artères coronaires de longueur standard. Par ailleurs, l'utilisation d'un fil-guide 3 comportant des capteurs 31 et 32 maintenus à une distance prédéfinie permet d'éliminer les imprécisions liées à la distance entre deux mesures de pression à l'intérieur d'une artère coronaire.

À l'opposée de son extrémité libre, le fil 39 est fixé à une poignée de préhension 36. Le fourreau 33 et la gaine 34 sont ici encastrés dans la poignée de préhension 36. La poignée 36 permet ainsi de déplacer le fil 39. Dans cet exemple, le fil-guide 3 est configuré pour fournir les signaux de pression mesurés à un système de traitement, par l'intermédiaire d'une interface sans fil. On peut cependant également envisager que le fil-guide 3 communique avec un système de traitement par une interface câblée. Un circuit de numérisation et de pilotage 38 est ici logé à l'intérieur de la poignée 36. Les câbles ou fibres optiques 311 et 321 du fil 39 sont connectés au circuit 38. Le circuit 38 est connecté à une antenne émettrice 37. Le circuit 38 est configuré pour numériser les signaux mesurés par les capteurs 31 et 32 et fournis par les câbles ou fibres optiques 311 et 321. Le circuit 38 est également configuré pour transmettre les signaux numérisés à distance, par l'intermédiaire de l'antenne 37, par un protocole de communication approprié. L'alimentation électrique du circuit 38 se fait de façon connue en soi et ne sera pas décrite ici.

La gaine 34 peut être réalisée en matériau hydrophobe au niveau de l'extrémité libre du fil 39, et peut être réalisée dans un autre matériau tel que du PTFE (polytétrafluoroéthylène) entre l'extrémité libre et la poignée 36.

L'utilisation d'un fil-guide de FFR 3, dont l'utilisation est validée par les autorités de santé et fait partie de la routine clinique, permet d'utiliser un système 4 selon l'invention avec un processus de validation clinique sensiblement allégé.

Le fil-guide 3 communique avec un système de traitement de signaux 4. Le système 4 comprend ici une interface de réception ou communication 41 avec le fil-guide 3 de type sans fil. On peut cependant également envisager que le fil-guide 3 communique avec un système de traitement 42 par une interface câblée.Le système 4 comprend ainsi une antenne de réception formant interface de réception 41, configurée pour recevoir les informations communiquées par l'antenne 37. L'antenne de réception 41 est connectée à un circuit de traitement 42, par exemple un ordinateur. Le système 4 comprend une interface de communication filaire 43. L'interface 43 permet par exemple d'afficher les résultats calculés par le circuit de traitement 42 sur un écran d'affichage 5. Un filtre anti-repliement et un convertisseur analogique/numérique peuvent par exemple être intégrés dans le circuit de traitement 42, soit dans le fil-guide 3, afin de permettre au circuit de traitement 42 de réaliser des traitements sur des signaux numériques de pressions sanguines coronaires proximale et distale.

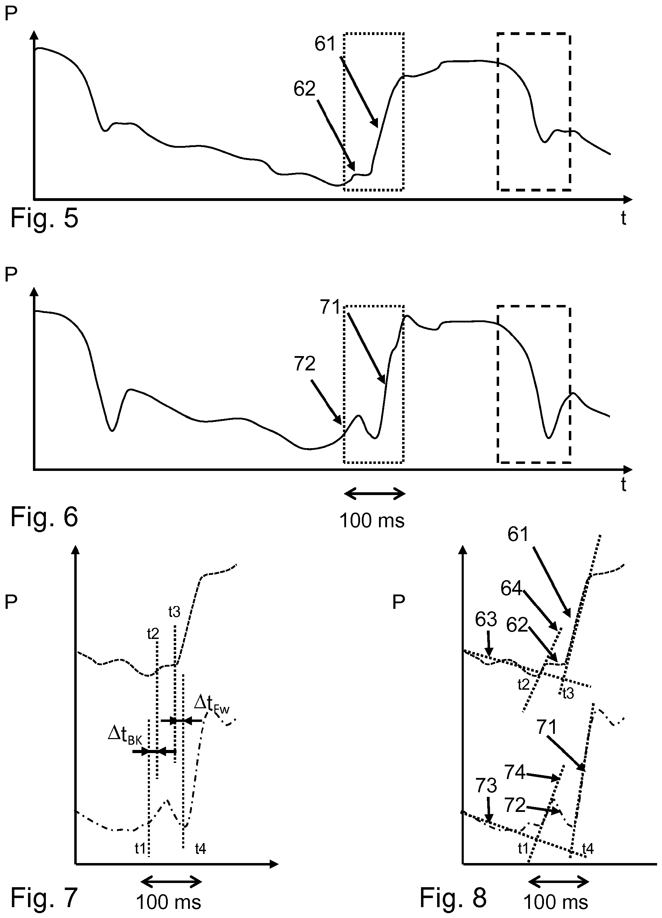

La figure 5 est un diagramme illustrant un exemple de cycle de pression artérielle coronaire proximale et la figure 6 est un diagramme illustrant un exemple de cycle de pression artérielle coronaire distale. Lors d'une phase de compression, illustrée dans la fenêtre en pointillés, les pressions artérielles passent d'une valeur de pression diastolique à une valeur de pression systolique. Durant la phase de compression, la pression proximale comporte un front montant 61, précédé d'un pic de pression 62. Le pic de pression 62 présente une amplitude inférieure à l'amplitude du front montant 61 (cette dernière amplitude étant égale à la pression systolique proximale moins la pression diastolique proximale). Lors d'une phase de décompression, illustrée dans la fenêtre en trait discontinu, les pressions artérielles proximales passent d'une valeur de pression systolique à une valeur de pression plus basse avec un nadir au moment de la fermeture de la valve aortique, moment de l'apparition de l'onde dicrote. Du côté distal de l'artère coronaire, durant la phase de compression, la pression distale comporte un front montant 71, précédé d'un pic de pression 72. Le pic de pression 72 présente une amplitude inférieure à l'amplitude du front montant 71 (cette dernière amplitude étant égale à la pression systolique distale moins la pression diastolique distale). Lors d'une phase de décompression, illustrée dans la fenêtre en trait discontinu, les pressions artérielles distales passent d'une valeur de pression systolique à une valeur de pression plus basse avec un nadir au moment de la fermeture de la valve aortique, moment de l'apparition de l'onde dicrote.

La figure 7 illustre des paramètres temporels au niveau du front montant d'une pression artérielle coronaire proximale et d'une pression artérielle coronaire distale. A partir des signaux de pression artérielle mesurés en position proximale (courbe du haut) et en position distale (courbe du bas), on peut faire ressortir des paramètres temporels. On constate que le pic de pression 72 débute à l'instant t1, le pic de pression 62 débute à l'instant t2, le front montant 61 débute à l'instant t3 et le front montant 71 débute à l'instant t4. On constate que l'instant t1 précède l'instant t2 d'une valeur Δtbk. On constate que l'instant t3 précède l'instant t4 d'une valeur Δtfw.

La figure 8 illustre un exemple d'extrapolation des courbes de pressions jusqu'aux instants t1 à t4 qui peut être effectué par le dispositif de traitement 42, à partir des signaux de pression artérielle. L'instant t2 est par exemple déterminé comme l'instant correspondant à l'intersection entre une droite (ou en alternative une courbe exponentielle, ou selon une autre loi) représentative de la baisse de pression diastolique (droite 63) et une droite 64 correspondant à la montée de pression du pic 62. L'instant t3 est par exemple déterminé comme l'instant correspondant à l'intersection entre la droite 63 et la droite correspondant au front montant 61. L'instant t1 est par exemple déterminé comme l'instant correspondant à l'intersection entre une droite (ou en alternative une courbe exponentielle ou selon une autre loi) représentative de la baisse de pression diastolique (droite 73) et une droite 74 correspondant à la montée de pression du pic 72. L'instant t4 est par exemple déterminé comme l'instant correspondant à l'intersection entre la droite 73 et la droite correspondant au front montant 71. Comme le pic distal 72 est en avance de phase sur le pic proximal 62, on est bien en présence d'une onde de pouls coronaire régressive, dont la vitesse est égale à Dmd/Δtbk. La vitesse de l'onde de pouls progressive, déterminée par l'écart entre le front proximal 61 et le front distal 71, est égale à Dmd/Δtfw. Selon l'invention, la vitesse d'onde de pouls est basée sur l'onde de pouls régressive.

Lors d'une étude effectuée sur des cobayes animaux sains (porc anesthésié) on a constaté que la vitesse d'onde de pouls progressive et la vitesse d'onde de pouls régressive sont fortement corrélées (r²=0.83,n=10) en état de base (pression artérielle et fréquence cardiaque spontanées). En présence d'une sténose coronaire (gonflement d'un ballonnet d'angioplastie entre les positions proximale et distale avec une aire de section transversale approximativement égale à la moitié de l'aire de section transversale de l'artère mesurée par la technique IVUS pour Intra-Vascular Ultra Sound en langue anglaise, soit technique d'ultrasons intra vasculaire), il s'avère que le calcul de la vitesse d'onde de pouls basée sur l'onde de pouls régressive est plus fiable que celle calculée sur l'onde de pouls progressive. On a par ailleurs constaté que le rapport entre l'amplitude de l'onde de pouls régressive et l'onde de pouls progressive augmentait avec la sévérité d'une sténose. Plus cette sténose est sévère et importante, plus le calcul de vitesse de pouls sur l'onde progressive est faussé, et plus le calcul de vitesse de pouls sur l'onde régressive est précis. Ainsi, le niveau de précision d'un système de calcul de vitesse d'onde de pouls selon l'invention s'avère d'autant plus précis que la pathologie est sévère.

Le fonctionnement du système de détermination de la vitesse d'onde de pouls 4 va maintenant être détaillé. L'interface de réception 41 est configurée pour recevoir le signal de pression sanguine proximale et le signal de pression sanguine distale pour une artère, soit en post traitement, soit en provenance des capteurs 31 et 32.

Le dispositif de traitement 42 est configuré, de façon connue en soi, pour déterminer un front montant proximal entre une pression diastolique et une pression systolique du signal de pression sanguine proximale. Le front montant proximal correspond à une montée de pression proximale entre la pression diastolique proximale et la pression systolique proximale. Le dispositif de traitement 42 est ainsi configuré pour déterminer l'instant t3 détaillé auparavant. Le dispositif de traitement 42 est également configuré, de façon connue en soi, pour déterminer un front montant distal entre une pression diastolique et une pression systolique du signal de pression sanguine distale. Le front montant distal correspond à une montée de pression distale entre la pression diastolique distale et la pression systolique distale. Le dispositif de traitement 42 est ainsi configuré pour déterminer l'instant t4 détaillé auparavant.

On peut par exemple envisager d'échantillonner une pression distale et/ou une pression proximale à une fréquence comprise entre 500 Hz et 5 kHz. Pour une fréquence d'échantillonnage jugée insuffisante, on peut procéder à une interpolation des valeurs d'échantillonnage (par exemple par utilisation de splines cubiques), puis à un nouvel échantillonnage du signal interpolé à une fréquence supérieure à la fréquence d'échantillonnage initiale (sur-échantillonnage). Par exemple, pour une fréquence d'échantillonnage de 500 Hz, on peut envisager un sur-échantillonnage du signal interpolé à une fréquence de 2 kHz ou plus.

Le dispositif de traitement 42 est également configuré pour déterminer le pic de pression proximal 62 préalable au front montant proximal 61, durant une phase de baisse de pression diastolique proximale. Le dispositif de traitement 42 est ainsi configuré pour déterminer l'instant t2 détaillé auparavant. Le dispositif de traitement 42 est encore configuré pour déterminer le pic de pression distal 72 préalable au front montant distal 71, durant une phase de baisse de pression diastolique distale. Le dispositif de traitement 42 est ainsi configuré pour déterminer l'instant t1 détaillé auparavant. Le dispositif de traitement 42 pourra être configuré pour rechercher un pic de pression dans une fenêtre temporelle d'une durée entre 50 et 100 ms avant le front montant correspondant.

Le dispositif de traitement 42 est également configuré pour déterminer l'amplitude des pics de pression. Si plusieurs pics de pression sont identifiés dans cette fenêtre temporelle, le dispositif de traitement 42 sélectionne le pic de pression présentant l'amplitude la plus élevée. L'identification d'un pic de pression peut être conditionnée par un pic présentant une amplitude supérieure à un seuil fixe ou supérieure à une proportion prédéfinie de la pression pulsée (différence entre la pression systolique et la pression diastolique).

Le dispositif de traitement 42 détermine ensuite la vitesse de propagation de l'onde de pouls régressive en fonction d'une avance de phase du pic de pression distal par rapport au pic de pression proximal déterminés. En particulier, la vitesse VOPr de propagation de l'onde de pouls régressive peut utiliser la relation suivante : VOPr=(t2-t1 )/Dmd cette relation est basée sur l'exploitation d'une référence temporelle reçue sur l'interface de réception 41 pour le signal de pression sanguine proximale et pour le signal de pression sanguine distale respectivement.

La distance Dmd peut être soit une valeur fixe correspondant à une distance prédéterminée entre les capteurs 31 et 32 (valeur par exemple mémorisée dans le fil guide 3 ou dans le système 4), soit une valeur de déplacement d'un unique capteur, pour lesquels des mesures de pression sont réalisées séquentiellement et séparées par la distance Dmd. On peut également prévoir d'utiliser un fil-guide de FFR muni d'un unique capteur de pression, déplacé par le praticien d'une distance prédéfinie entre la position distale et la position proximale dans l'artère étudiée. Lors de l'analyse des signaux de pression respectifs en position proximale et en position distale, cette distance Dmd est prise en compte pour le calcul de la vitesse d'onde de pouls.

L'interface de réception 41 peut également être configurée pour recevoir un indicateur temporel d'un événement de synchronisation choisi parmi une contraction cardiaque isovolumique et une ouverture de valve aortique, pour le cœur raccordé à l'artère à analyser. L'interface de réception 41 peut également être configurée pour recevoir un signal d'électrocardiogramme, un signal sonore ou un signal d'imagerie pour le cœur raccordé à l'artère à analyser. Ainsi, dans le cas où les signaux de pression proximale et de pression distale ne sont pas simultanés, ils pourront être synchronisés avec un signal de référence commun ou un événement de synchronisation commun pour le cœur du patient.

Lorsque le dispositif de traitement 42 n'identifie pas de pic de pression préalable à son front montant respectif, il met en œuvre un calcul de vitesse d'onde de pouls basé sur l'onde de pouls progressive, par exemple comme détaillé dans le document EP3251591.

Avantageusement, le dispositif de traitement 42 peut être configuré pour recevoir une information de position du lieu de mesure de pression dans l'artère. Le dispositif de traitement 42 peut alors être configuré pour déterminer une position de référence pour le capteur de pression, à partir de laquelle les ondes régressives apparaissent ou disparaissent. Le dispositif 42 peut être configuré pour calculer la vitesse d'onde régressive pour plusieurs positions à partir de la position de référence. Le dispositif 42 pourra sélectionner ou retenir la valeur de vitesse d'onde régressive calculée pour la position la plus distante de la position de référence.

Le dispositif de traitement 42 peut déterminer les instants t3 et t4 par d'autres procédés que ceux décrits auparavant. En particulier, le dispositif de traitement 42 peut calculer la dérivée première ou seconde d'une pression proximale et/ou distale, puis déterminer les instants auquel cette dérivée première ou seconde franchit un seuil positif et un seuil négatif respectivement, pour identifier le front correspondant. Le dispositif de traitement 42 peut déterminer les instants t1 et t2 par d'autres procédés que ceux décrits auparavant. En particulier, le dispositif de traitement 42 peut calculer la dérivée première ou seconde d'une pression proximale et/ou distale, puis déterminer les instants auquel cette dérivée première ou seconde franchit un seuil positif et un seuil négatif respectivement pour identifier le pic de pression correspondant.

Avantageusement, le circuit 42 peut mettre en œuvre un filtrage passe-bas (par exemple avec une fréquence de coupure entre 10 et 20 Hz), pour éliminer les fluctuations rapides de pression entre les battements cardiaques, avant de déterminer la présence des pics de pression et les instants de leur apparition.

La vitesse d'onde de pouls régressive calculée peut être comparée à un seuil de référence pour une artère et un patient similaires. Lorsque la vitesse d'onde de pouls régressive calculée franchit un tel seuil de référence (le cas échéant un seuil bas ou un seuil haut), le circuit de traitement 42 pourra générer un signal d'alerte approprié pour attirer l'attention d'un praticien. Différents seuils pourront être utilisés, notamment en fonction de différents facteurs de risque, tels que l'hypertension, le diabète, la dyslipidémie, le tabagisme, l'hérédité de problèmes cardiovasculaires coronaires, un épisode cardiovasculaire coronaire antérieur, ou la composition de la plaque d'athérome estimée à partir des méthodes d'imagerie médicale.

## Revendications

1. [Système de détermination d'une vitesse d'onde de pouls (4), comprenant :
- une interface de réception (41) d'un signal de pression sanguine proximale dans une artère (12, 13, 14, 15) et de réception d'un signal de pression sanguine distale dans cette artère;
- un dispositif de traitement (42) configuré pour :
- déterminer un front montant proximal entre une pression diastolique et une pression systolique du signal de pression sanguine proximale ;
- déterminer un front montant distal entre une pression diastolique et une pression systolique du signal de pression sanguine distale ;
**caractérisé en ce que** le dispositif de traitement (42) est en outre configuré pour :
- déterminer un pic de pression proximal préalable audit front montant proximal ;
- déterminer un pic de pression distal préalable audit front montant distal, et déterminer que le pic de pression distal est en avance de phase par rapport au pic de pression proximal ;
- déterminer une vitesse de propagation d'une onde de pouls régressive en fonction de l'avance de phase du pic de pression distal par rapport au pic de pression proximal.

2. Système selon la revendication 1, dans lequel ladite interface de réception (41) est configurée pour recevoir une référence temporelle pour le signal de pression sanguine proximale et pour le signal de pression sanguine distale, ledit dispositif de traitement (42) étant configuré pour déterminer la vitesse de propagation de l'onde de pouls régressive en fonction du décalage temporel entre le pic de pression distal et le pic de pression proximal.

3. Système selon la revendication 1 ou 2, dans lequel ladite interface de réception (41) est configurée pour recevoir un indicateur temporel d'un événement de synchronisation choisi parmi une contraction cardiaque isovolumique et une ouverture de valve aortique du cœur raccordé à ladite artère.

4. Système selon l'une quelconque des revendications précédentes, dans lequel l'interface de réception (41) est configurée pour recevoir un signal d'électrocardiogramme, un signal sonore ou un signal d'imagerie du cœur raccordé à ladite artère.

5. Système selon l'une quelconque des revendications précédentes, dans lequel ladite interface de réception (41) est en outre configurée pour recevoir la position d'un capteur de pression (31,32), le dispositif de traitement (42) étant configuré pour déterminer une position de référence du capteur de pression pour laquelle les ondes régressives disparaissent.

6. Système selon l'une quelconque des revendications précédentes, dans lequel le dispositif de traitement (42) est en outre configuré pour déterminer l'amplitude du pic de pression distal.

7. Système selon la revendication 6, dans lequel le dispositif de traitement (42) détermine la présence du pic de pression distal lorsque l'amplitude de celui-ci dépasse un seuil prédéfini.

8. Système selon la revendication 6 ou 7, dans lequel ledit dispositif de traitement est en outre configuré pour calculer le rapport entre l'amplitude du pic de pression distal et le front montant distal.

9. Système selon l'une quelconque des revendications précédentes, dans lequel ledit dispositif de traitement (42) est configuré pour :
- identifier des phases de baisse de pression diastolique respectives dans le signal de pression sanguine proximale et dans le signal de pression sanguine distale ;
- identifier le début des fronts montants des pressions distale et proximale, en déterminant l'intersection entre les phases de baisse de pression diastolique identifiées et des tangentes respectives aux fronts montants des pressions distale et proximale.

10. Système selon l'une quelconque des revendications précédentes, dans lequel ledit dispositif de traitement (42) est configuré pour :
- identifier des phases de baisse de pression diastolique respectives dans le signal de pression sanguine proximale et dans le signal de pression sanguine distale ;
- identifier le début des pics des pressions distale et proximale, en déterminant l'intersection entre les phases de baisse de pression diastolique identifiées et des tangentes respectives aux pics de pressions distale et proximale.

11. Système selon l'une quelconque des revendications précédentes, dans lequel ladite interface de réception (41) est configurée pour récupérer la valeur de la distance entre le lieu de mesure de la pression proximale et le lieu de mesure de la pression distale, ledit dispositif de traitement (42) étant configuré pour déterminer ladite vitesse de propagation de l'onde de pouls régressive en fonction de la valeur de la distance récupérée.

12. Système selon l'une quelconque des revendications précédentes, comprenant en outre un fil-guide allongé de FFR (3), comportant deux capteurs de pression (31,32) décalés d'une distance prédéfinie sur la longueur du fil-guide (3), lesdits deux capteurs de pression étant connectés à ladite interface de réception (41), ladite interface de réception comprenant un circuit d'échantillonnage des signaux respectifs desdits capteurs de pression.

13. Système selon les revendications 5 et 12 en combinaison, dans lequel ladite interface de réception (41) est configurée pour recevoir une information de position desdits capteurs de pression dans une artère, ledit dispositif de traitement (42) étant configuré pour mémoriser la vitesse de propagation de l'onde de pouls régressive pour plusieurs positions desdits capteurs distantes de ladite position de référence déterminée, ledit dispositif de traitement (42) étant configuré pour sélectionner la vitesse de propagation de l'onde de pouls régressive pour la position desdits capteurs la plus distante de ladite position de référence déterminée.

## Patentansprüche

1. System zur Bestimmung der Geschwindigkeit einer Pulswelle (4), umfassend:
- eine Empfangsschnittstelle (41) für ein Signal eines proximalen Blutdrucks in einer Arterie (12, 13, 14, 15) und für ein Signal eines distalen Blutdrucks in dieser Arterie;
- eine Behandlungsvorrichtung (42), die zu Folgendem konfiguriert ist:
- Bestimmen einer proximalen Anstiegsflanke zwischen einem diastolischen Druck und einem systolischen Druck des proximalen Blutdrucksignals;
- Bestimmen einer distalen Anstiegsflanke zwischen einem diastolischen Druck und einem systolischen Druck des distalen Blutdrucksignals;
**dadurch gekennzeichnet, dass** die Behandlungsvorrichtung (42) außerdem zu Folgendem konfiguriert ist;
- Bestimmen einer Spitze des proximalen Drucks vor der proximalen Anstiegsflanke;
- Bestimmen einer Spitze des distalen Drucks vor der distalen Anstiegsflanke und Bestimmen, dass die Spitze des distalen Drucks gegenüber der Spitze des proximalen Drucks in der Phase voreilt;
- Bestimmen einer Fortpflanzungsgeschwindigkeit einer regressiven Pulswelle in Abhängigkeit von der Phasenvoreilung der Spitze des distalen Drucks gegenüber der Spitze des proximalen Drucks.

2. System nach Anspruch 1, wobei die Empfangsschnittstelle (41) dazu konfiguriert ist, eine Zeitreferenz für das proximale Blutdrucksignal und für das distale Blutdrucksignal zu empfangen, wobei die Behandlungsvorrichtung (42) dazu konfiguriert ist, die Fortpflanzungsgeschwindigkeit der regressiven Pulswelle in Abhängigkeit vom Zeitversatz zwischen der Spitze des distalen Drucks und der Spitze des proximalen Drucks zu bestimmen.

3. System nach Anspruch 1 oder 2, wobei die Empfangsschnittstelle (41) dazu konfiguriert ist, einen Zeithinweis auf ein Synchronisationsereignis zu empfangen, das aus einer isovolumetrischen Herzkontraktion und einer Öffnung der Aortenklappe des Herzens ausgewählt ist, das mit der Arterie verbunden ist.

4. System nach einem der vorhergehenden Ansprüche, wobei die Empfangsschnittstelle (41) dazu konfiguriert ist, ein Elektrokardiogrammsignal, ein Tonsignal oder ein Bildsignal des Herzens zu empfangen, das mit der Arterie verbunden ist.

5. System nach einem der vorhergehenden Ansprüche, wobei die Empfangsschnittstelle (41) außerdem dazu konfiguriert ist, die Position eines Drucksensors (31, 32) zu empfangen, wobei die Behandlungsvorrichtung (42) dazu konfiguriert ist, eine Referenzposition des Drucksensors zu bestimmen, bei der die regressiven Wellen verschwinden.

6. System nach einem der vorhergehenden Ansprüche, wobei die Behandlungsvorrichtung (42) außerdem dazu konfiguriert ist, die Amplitude der Spitze des distalen Drucks zu bestimmen.

7. System nach Anspruch 6, wobei die Behandlungsvorrichtung (42) das Vorhandensein der Spitze des distalen Drucks bestimmt, wenn deren Amplitude einen vorgegebenen Schwellenwert überschreitet.

8. System nach Anspruch 6 oder 7, wobei die Behandlungsvorrichtung außerdem dazu konfiguriert ist, das Verhältnis zwischen der Amplitude der Spitze des distalen Drucks und der distalen Anstiegsflanke zu berechnen.

9. System nach einem der vorhergehenden Ansprüche, wobei die Behandlungsvorrichtung (42) zu Folgendem konfiguriert ist:
- Erkennen der jeweiligen Phasen einer Absenkung des diastolischen Drucks im proximalen Blutdrucksignal und im distalen Blutdrucksignal;
- Erkennen des Anfangs der Anstiegsflanken des distalen und proximalen Drucks, indem der Schnittpunkt zwischen den erkannten Phasen der Absenkung des diastolischen Drucks und den jeweiligen Tangenten an den Anstiegsflanken des distalen und proximalen Drucks bestimmt wird.

10. System nach einem der vorhergehenden Ansprüche, wobei die Behandlungsvorrichtung (42) zu Folgendem konfiguriert ist:
- Erkennen der jeweiligen Phasen der Absenkung des diastolischen Drucks im proximalen Blutdrucksignal und im distalen Blutdrucksignal;
- Erkennen des Anfangs der Spitzen des distalen und proximalen Drucks, indem der Schnittpunkt zwischen den erkannten Phasen der Absenkung des diastolischen Drucks und den jeweiligen Tangenten an den Spitzen des distalen und proximalen Drucks bestimmt wird.

11. System nach einem der vorhergehenden Ansprüche, wobei die Empfangsschnittstelle (41) dazu konfiguriert ist, den Wert des Abstands zwischen dem Messort des proximalen Drucks und dem Messort des distalen Drucks zu erhalten, wobei die Behandlungsvorrichtung (42) dazu konfiguriert ist, die Fortpflanzungsgeschwindigkeit der regressiven Pulswelle in Abhängigkeit von dem Wert des erhaltenen Abstands zu bestimmen.

12. System nach einem der vorhergehenden Ansprüche, außerdem umfassend einen langgestreckten FFR-Führungsdraht (3), umfassend zwei Drucksensoren (31, 32) die um einen vorgegebenen Abstand auf der Länge des Führungsdrahts (3) versetzt sind, wobei die zwei Drucksensoren mit der Empfangsschnittstelle (41) verbunden sind, wobei die Empfangsschnittstelle einen Schaltkreis zur Abtastung der jeweiligen Signale der Drucksensoren umfasst.

13. System nach den Ansprüchen 5 und 12 in Kombination, wobei die Empfangsschnittstelle (41) dazu konfiguriert ist, eine Information über die Position der Drucksensoren in einer Arterie zu empfangen, wobei die Behandlungsvorrichtung (42) dazu konfiguriert ist, die Fortpflanzungsgeschwindigkeit der regressiven Pulswelle für mehrere, von der bestimmten Referenzposition entfernte Positionen der Sensoren zu speichern, wobei die Behandlungsvorrichtung (42) dazu konfiguriert ist, die Fortpflanzungsgeschwindigkeit der regressiven Pulswelle für die Position der Sensoren auszuwählen, die von der bestimmten Referenzposition am entferntesten ist.

## Claims

1. System for determining a pulse wave velocity (4), comprising:
- an interface (41) for receiving a signal of proximal blood pressure in an artery (12, 13, 14, 15) and for receiving a signal of distal blood pressure in this artery;
- a processing device (42) configured to:
- determine a proximal rising edge between a diastolic pressure and a systolic pressure of the proximal-blood-pressure signal;
- determine a distal rising edge between a diastolic pressure and a systolic pressure of the distal-blood-pressure signal;
**characterized in that** the processing device (42) is further configured to:
- determine a proximal pressure peak prior to said proximal rising edge;
- determine a distal pressure peak prior to said distal rising edge, and to determine whether the distal pressure peak is in phase advance with respect to the proximal pressure peak;
- determine a propagation velocity of a backward pulse wave depending on the phase advance of the distal pressure peak with respect to the proximal pressure peak.

2. System according to Claim 1, wherein said receiving interface (41) is configured to receive a time reference for the proximal-blood-pressure signal and for the distal-blood-pressure signal, said processing device (42) being configured to determine the propagation velocity of the backward pulse wave depending on the temporal offset between the distal pressure peak and the proximal pressure peak.

3. System according to Claim 1 or 2, wherein said receiving interface (41) is configured to receive a time indicator of a synchronization event selected from an isovolumic cardiac contraction and opening of the aortic valve of the heart connected to said artery.

4. System according to any preceding claim, wherein the receiving interface (41) is configured to receive an electrocardiogram signal, an audio signal or an imaging signal from the heart connected to said artery.

5. System according to any preceding claim, wherein said receiving interface (41) is further configured to receive the position of a pressure sensor (31, 32), the processing device (42) being configured to determine a reference pressure-sensor position in which the backward waves disappear.

6. System according to any preceding claim, wherein the processing device (42) is further configured to determine the amplitude of the distal pressure peak.

7. System according to Claim 6, wherein the processing device (42) determines the presence of the distal pressure peak when the amplitude thereof exceeds a predefined threshold.

8. System according to Claim 6 or 7, wherein said processing device is further configured to compute the ratio between the amplitude of the distal pressure peak and the distal rising edge.

9. System according to any preceding claim, wherein said processing device (42) is configured to:
- identify respective phases of decrease in diastolic pressure in the proximal-blood-pressure signal and in the distal-blood-pressure signal;
- identify the beginning of the rising edges of the distal and proximal pressures, by determining the intersection between the identified phases of decrease in diastolic pressure and respective tangents to the rising edges of the distal and proximal pressures.

10. System according to any preceding claim, wherein said processing device (42) is configured to:
- identify respective phases of decrease in diastolic pressure in the proximal-blood-pressure signal and in the distal-blood-pressure signal;
- identify the beginning of the peaks of the distal and proximal pressures, by determining the intersection between the identified phases of decrease in diastolic pressure and respective tangents to the peaks of the distal and proximal pressures.

11. System according to any preceding claim, wherein said receiving interface (41) is configured to retrieve the value of the distance between the site of measurement of the proximal pressure and the site of measurement of the distal pressure, said processing device (42) being configured to determine said propagation velocity of the backward pulse wave depending on the retrieved value of the distance.

12. System according to any preceding claim, further comprising an elongate FFR guidewire (3) comprising two pressure sensors (31, 32) that are offset by a predefined distance along the length of the guidewire (3), said two pressure sensors being connected to said receiving interface (41), said receiving interface comprising a circuit for sampling the respective signals of said pressure sensors.

13. System according to Claims 5 and 12 in combination, wherein said receiving interface (41) is configured to receive information on the position of said pressure sensors in an artery, said processing device (42) being configured to store the propagation velocity of the backward pulse wave for a plurality of positions of said sensors away from said determined reference position, said processing device (42) being configured to select the propagation velocity of the backward pulse wave for the position of said sensors that is furthest away from said determined reference position.
